(19) Europäisches Patentamt
European Patent Office
Office européen des brevets

(11) EP 1 655 288 A1

(12) EUROPEAN PATENT APPLICATION

(43) Date of publication:
10.05.2006 Bulletin 2006/19

(21) Application number: 04292629.5

(22) Date of filing: 05.11.2004

(51) Int Cl.:
*C07D 239/54* (2006.01) *A61K 31/513* (2006.01)
*A61P 31/06* (2006.01) *A61P 31/08* (2006.01)

(84) Designated Contracting States:
AT BE BG CH CY CZ DE DK EE ES FI FR GB GR HU IE IS IT LI LU MC NL PL PT RO SE SI SK TR
Designated Extension States:
AL HR LT LV MK YU

(71) Applicants:
- INSTITUT PASTEUR
75724 Paris Cédex 15 (FR)
- INSTITUT NATIONAL DE LA SANTE ET DE LA RECHERCHE MEDICALE (INSERM)
75013 Paris (FR)
- CENTRE NATIONAL DE LA RECHERCHE SCIENTIFIQUE (CNRS)
75016 Paris (FR)

(72) Inventors:
- Munier-Lehmann, Hélène
92190 Meudon (FR)
- Labesse, Gilles
34000 Montpellier (FR)
- Douguet, Dominique
34070 Montpellier (FR)
- Pochet, Sylvie
75012 Paris (FR)

(74) Representative: Corizzi, Valérie et al
Cabinet ORES,
36, rue de Saint Pétersbourg
75008 Paris (FR)

(54) **Aryl pyrimidyl compounds, pharmaceutical compositions comprising them, their use as antimicrobial agents**

(57) Molecule responding to formula (I):

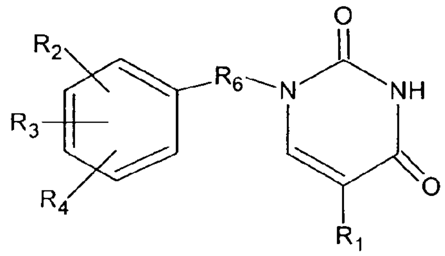

(I)

and its use for the preparation of a medicament for the prevention and/or treatment of a pathology caused by a mycobacteria

EP 1 655 288 A1

## Description

The present invention relates to certain substituted aryl pyrimidyl compounds and to a process for their synthesis. It also relates to pharmaceutical compositions comprising them and to their use as antimicrobial agents, especially for the prevention or treatment of pathologies in relationship with a mycobacteria.

The invention relates in particular to the use of such molecules for the prevention or treatment of tuberculosis and other diseases caused by a mycobacteria.

The incidence of tuberculosis has been increasing during the last twenty years and it is now the first cause of mortality among infectious diseases in the world, killing more than two million people a year. *Mycobacterium tuberculosis* (*M.tuberculosis*) is the principal microbial agent involved for humans. Tuberculosis is primarily transmitted via airborne aerosoled secretions. A peculiar aspect of its pathogenicity comes from the fact that it can remain quiescent and become active decades later. One of the most significant risk factor for developing tuberculosis is human immunodeficiency virus (HIV) infection. The current treatment of active tuberculosis includes four drugs (isoniazid, rifampicin, pyrazinamide and ethambutol) for at least six months. A significant proportion of patients do not complete the therapy, especially in developing countries, and this has led to the appearance of resistant strains of *M. tuberculosis*.

Consequently, there is a need for new molecules which are efficient against *M. tuberculosis*.

In this context thymidine monophosphate kinase (TMPK), one essential enzyme of nucleotide metabolism is an interesting target.

TMPK (E.C.2.7.4.9, ATP:TMP phosphotransferase) belongs to a large superfamily of nucleoside monophosphate kinases (NMPK). It catalyses the phosphorylation of thymidine monophosphate (TMP) to thymidine diphosphate (TDP) utilizing ATP as its preferred phosphoryl donor. It lies at the junction of the *de novo* and salvage pathways of thymidine triphosphate (TTP) metabolism and is the last specific enzyme for its synthesis. These characteristics make TMPK a good target for the design of new antibiotic drugs.

Purine and pyrimidine nucleoside analogues acting on the TMPK of *M tuberculosis* have been disclosed in S. Pochet *et al.*, Chem. Bio. Chem. 2003, **4,** 742-747.

However there is always a need for molecules with a stronger biological activity, a better specificity, an improved bioavailability, and molecules which would be easier to synthesize, so that their production on industrial scale can be envisioned.

An object of the instant invention is the molecules responding to formula (I):

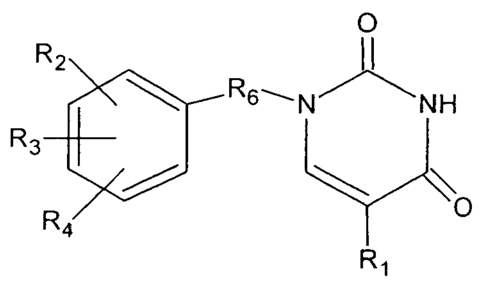

**(I)**

wherein:

- $R_1$ is selected from the group consisting of: $CH_3$, $-CF_3$, a halogen atom, $-NH_2$, , -COOH, $-CONH_2$,
- $R_2$, $R_3$, $R_4$, identical or different, are selected from the group consisting of: H, a halogen atom, $C_1$-$C_8$ alkyl, $C_2$-$C_8$ alkenyl, $C_2$-$C_8$ alkynyl, -OH, - $NH_2$, -COOH, $-CONH_2$, -CN, $-COOR_5$, $-COR_5$, $-OR_5$, substituted $C_1$-$C_8$ alkyl , substituted $C_2$-$C_8$ alkenyl, or substituted $C_2$-$C_8$ alkynyl wherein the substituent is selected from the group consisting of: -OH, $-NH_2$, -COOH, $-CONH_2$, -CN, $-COOR_5$, - $COR_5$, $-OR_5$, a halogen atom;
- $R_5$ is selected from the group consisting of $C_1$-$C_6$ alkyl;
- $R_6$ is selected among: $C_1$-$C_4$ alkyl, $C_2$-$C_4$ alkenyl, carbonyl (=C=O), $-(CF_2)n-$
- n is an integer selected from 1, 2, 3,

and their pharmaceutically acceptable salts.

Alkyl is a linear, branched or cyclic hydrogeno carbon radical.

Alkenyl is a linear, branched or cyclic hydrocarbyl radical comprising at least one double bond.

Alkynyl is a linear, branched or cyclic hydrocarbyl radical comprising at least one triple bond.

Halogen is selected from the group consisting of Cl, F, Br, I.

Preferentially, the molecule responding to formula (I) satisfies one or more of the following conditions.

- $R_6$ is $-CH_2-$;
- $R_1$ is selected from the group consisting of: $-CH_3$, -Br, -Cl;
- at least one group among $R_2$, $R_3$, $R_4$ is H.

More preferentially, $R_2 = R_3 = H$.

Advantageously $R_4$ is in the para position on the phenyl ring.

In a preferred manner, $R_4$ is selected from the group consisting of substituted $C_1$-$C_6$ alkyl or substituted $C_2$-$C_6$ alkenyl, wherein the substituent is -COOH.

Even more preferentially $R_4$ is selected from the group consisting of $C_2$-$C_4$ alkyl substituted by one -COOH.

Advantageously, $R_4$ is 4-yl-n-butyric acid

The favourite molecules are described by their chemical formula here-under:

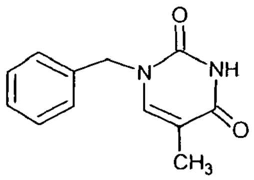

**3**

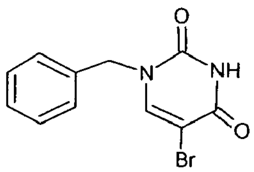

**4**

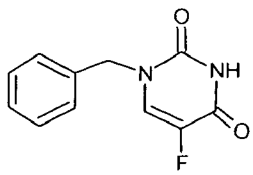

**5**

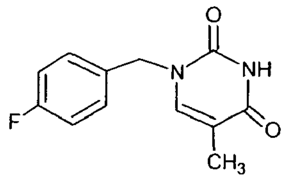

**6**

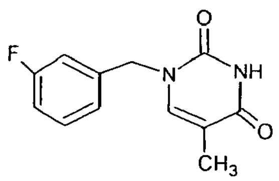

**7**

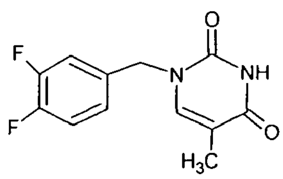

**8**

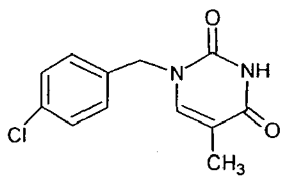

**9**

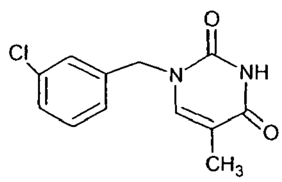

**10**

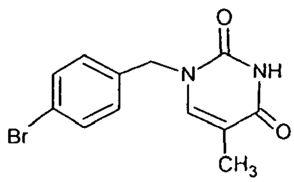

**11**

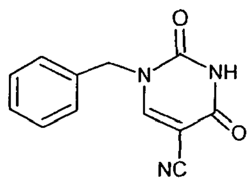

**12**

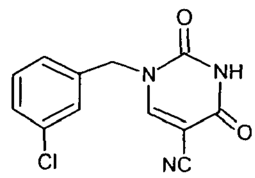

**13**

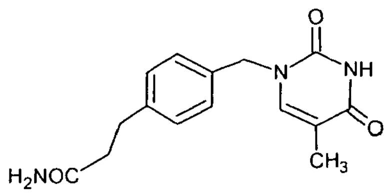

**1**

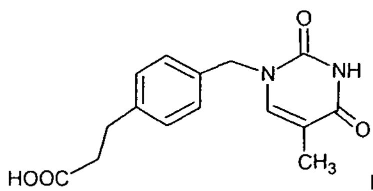

**2**

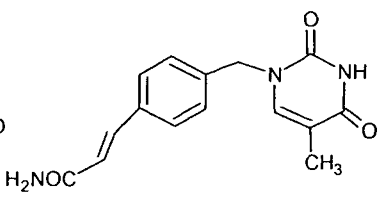

**14**

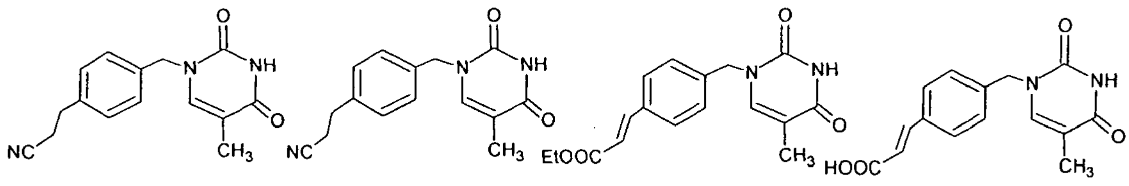

15 16 17 18

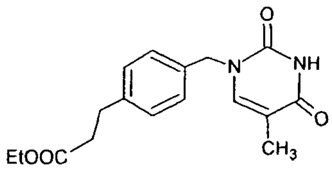

19

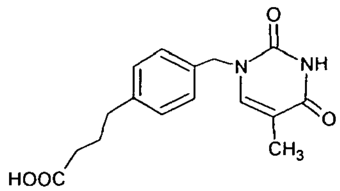

20

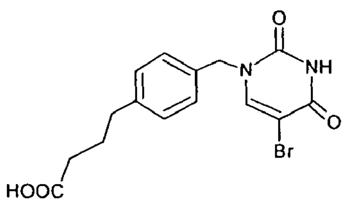

21

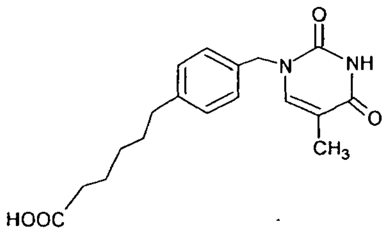

22

The above described molecules have demonstrated their capacity to inhibit *M tuberculosis TMPK* and consequently they can be used for the preparation of a medicament for the prevention and/or treatment of tuberculosis. It must be said that these molecules have a capacity to inhibit *M tuberculosis TMPK in vitro* which varies in Ki value, according to which molecule is concerned. However, the inhibitory activity exists and permits to have good hopes of an *in vivo* inhibition of *M. tuberculosis TMPK*. Preferred molecules are the ones whose Ki is inferior or equal to 40 $\mu$M, and even more preferentially inferior or equal to 30 $\mu$M. The molecules **20, 21** and **22** depicted here-above are the three favourite molecules for their action as inhibitors of *M. tuberculosis* TMPK.

The molecules of the invention can also be used as an inhibitor of a mycobacteria TMPK, especially *M. tuberculosis* TMPK *in vitro,* for biological tests for example.

Moreover, these molecules can also be used for the preparation of a medicament for the prevention or treatment of other pathologies caused by a mycobacteria, among which: leprosy (*M. leprae).*

The compounds of the instant invention have the advantage of having a lower affinity for human TMPK than for *M.tuberculosis* TMPK. Consequently, side effects of a drug based on these compounds administered at therapeutic dosage would be limited.

The instant invention encompasses pharmaceutical compositions comprising at least one compound of formula (I) in a pharmaceutically acceptable carrier. The routes of administration include the oral, buccal, intranasal, ocular, intraveneous, intramuscular, transdermal, parenteral and rectal routes. Preferentially when the pathology to be treated is tuberculosis, the pharmaceutical composition of the invention is administered by oral or intranasal route as tablets, pills, dragees, capsules, gels, suspensions, syrups. It may be distributed in an aerosol or as solution for inhalation or any other form which allows easy volatilization for rapid administration to the lung.

The dosage and posology of the pharmaceutical composition is adapted to the weight, age and condition of

the patient and to the inhibitory action of the molecule. The daily dose of active principle is comprised between 0,1 and 500 mg/kg.

Pharmaceutically acceptable salts encompass salts of acid functions of (I) with an organic or an inorganic base and salts of an amine function of (I) with an organic or mineral acid.

Among addition salts with acids are included: acetic, oxalic, succinic, fumaric, gluconic, malic, ascorbic, benzoic, hydrochloric, phosphoric, hydrobromic, sulphuric, sulfinic, formic, toluene sulfonic, methane sulfonic, nitric, benzoic, citric, tartaric, maleic, hydroiodic salts.

Among addition salts with bases are included : sodium, potassium and lithium salts; calcium, barium and magnesium salts; ammonium, ferrous, ferric, zinc, manganese, aluminium, magnesium salts; trimethylamine, triethylamine, tri(n-propyl)amine, dicyclohexylamine, triethanolamine, arginine, lysine, histidine, ethylenediamine, glucosamine, methylglucamine, purines, piperazines, piperidines, caffeine, procaine salts.

Pharmaceutically acceptable carriers can include one or several of the following compounds in a non limitating manner: fillers, such as sugar, starch, gelatine, gum, cellulose derivatives (methyl cellulose, hydroxy propylmethyl cellulose), polyvinylpyrrolidone, agar, alginic acid, talc, polyethylene glycol, pharmaceutically acceptable pigments and dyes, stabilizers, oils, liquid paraffin, ethanol, glycerids.

**PREPARATION OF MOLECULES**

Another object of the instant invention is a process for the synthesis of the molecules of formula (I).

This process can be described by scheme 1 here-under:

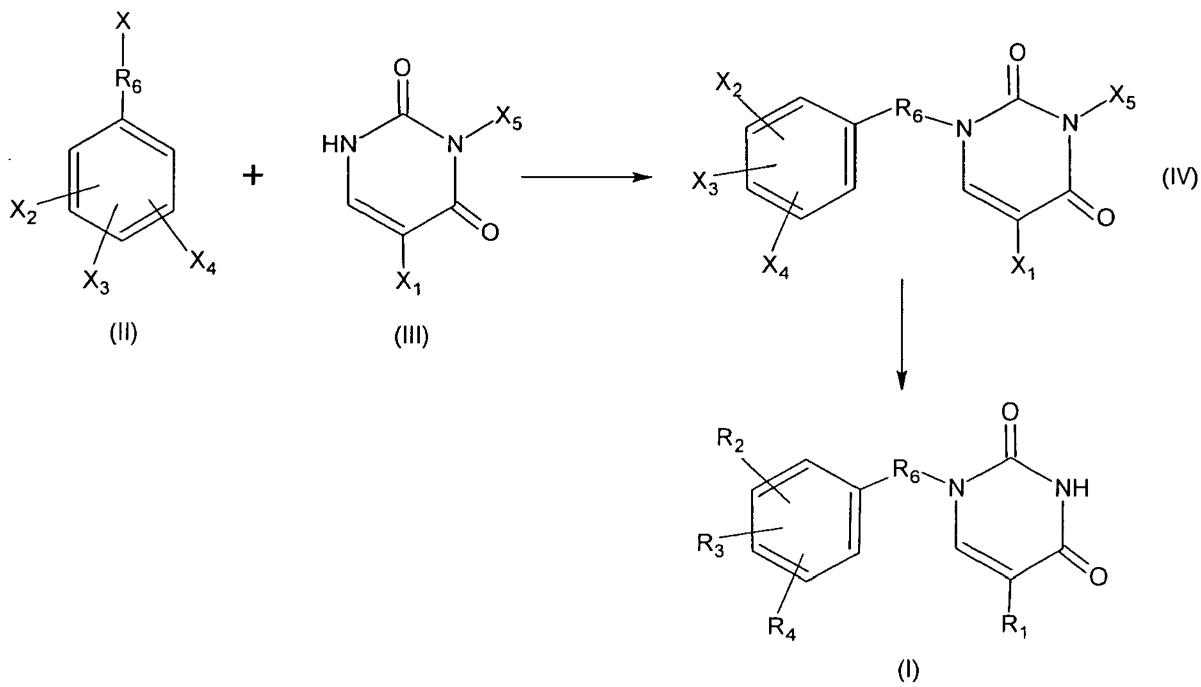

**Scheme 1**

According to the process of the instant invention a haloaryl of formula (II) is reacted with a thymine or thymine derivative or uracyle or uracyle derivative of formula (III) to give the condensate (IV). In formula (II): X represents a halogen atom, preferentially Br; $X_2$, $X_3$, $X_4$ are selected among $R_2$, $R_3$ and $R_4$ respectively and their chemical precursors. In formula (III) and in formula (IV), $X_1$ is selected among $R_1$ and its chemical precursors, $X_5$ is selected among H and the benzyl group (Bzl).

By chemical precursors ($X_1$, $X_2$, $X_3$ and $X_4$) is meant a functional group which can be transformed in one ore more steps into the desired functional group ($R_1$, $R_2$, $R_3$ and $R_4$).

As an example, if $R_1$ is H, or halogen, then $X_1$ is respectively H or said halogen, but if $R_1$ is $-CH_2-COOH$, then $X_1$ can be $-CH_2-COOBzl$, or $-CH_2-OH$, or $-CH_2-Br$, so that the alkylation of the thymine cycle can be effected in the absence of any side reaction.

In a second step and if necessary, $X_1$, $X_2$, $X_3$, $X_4$ and $X_5$ are transformed into $R_1$, $R_2$, $R_3$, $R_4$ and H respectively to give the molecule of formula (I).

According to a favourite variant of the invention, $R_2$=$R_3$=H and $R_6$=$CH_2$. Then a key intermediate molecule for the synthesis of the molecules of formula (I) is:

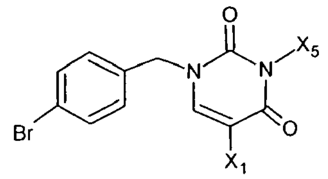

(V)

wherein $X_1$ and $X_5$ have the same definition as explained above.

And especially in the case when $R_1$ is $CH_3$, favourite intermediate molecules (V) for the preparation of other molecules of formula (I) are:

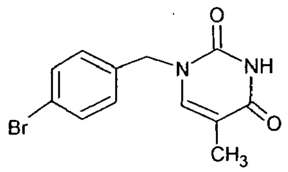

and

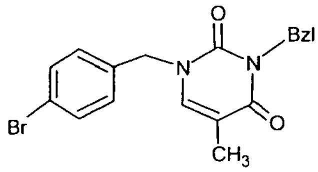

**11** **11bis**

The use of a compound of formula (V), and especially compounds **11** and **11bis** for the preparation of a molecule responding to formula (I) is another object of the invention.

The above-explained strategy is illustrated in the examples and in the schemes 2 and 3 here-under.

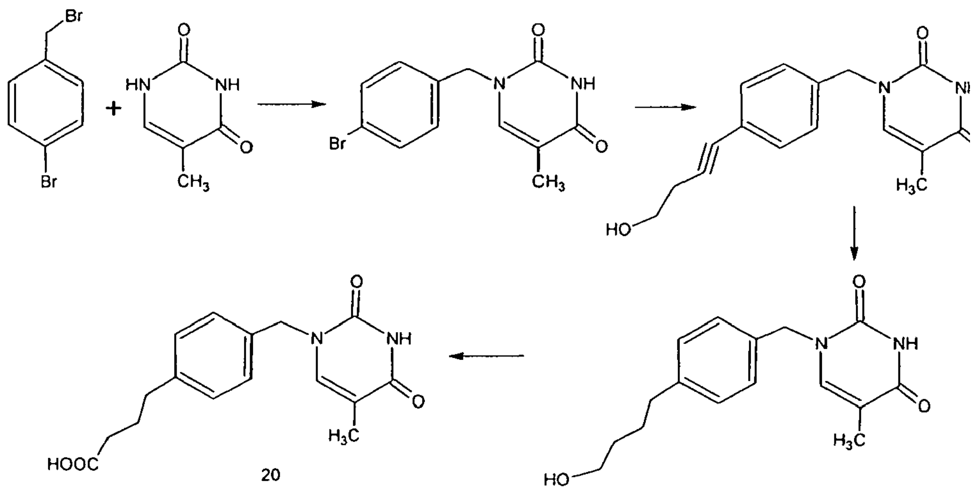

20

SCHEME 2

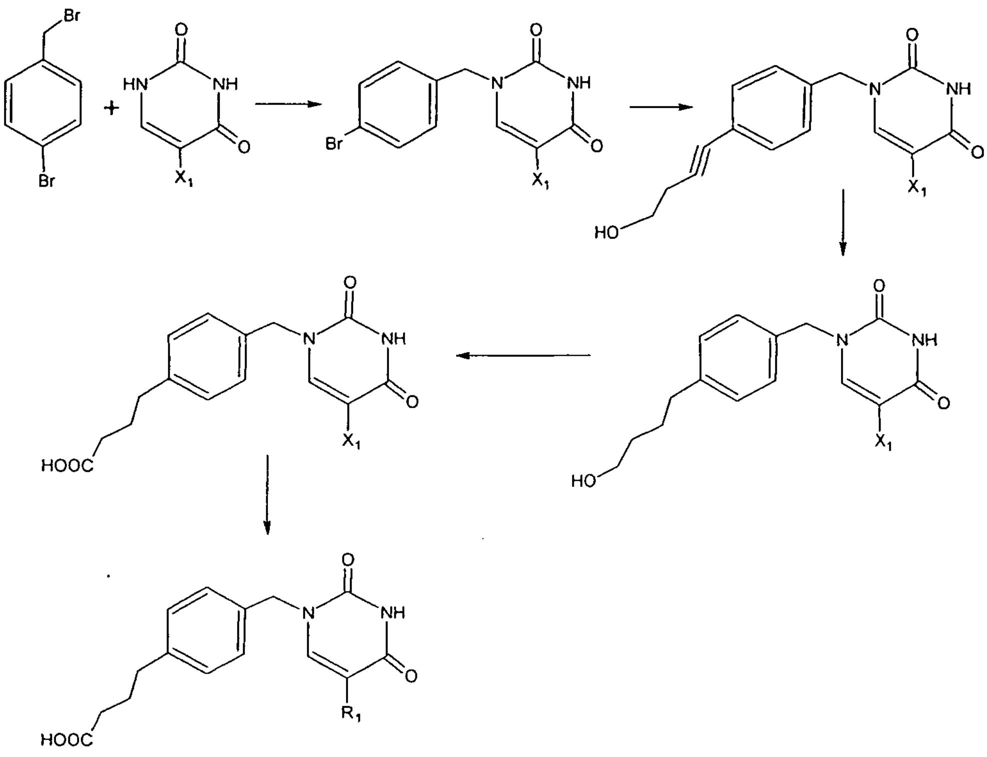

4.

$R_1$= Cl, Br $X_1$=H

SCHEME 3

**EXPERIMENTAL SECTION**

**A- Chemical Synthesis**

**General information** Solvents were spectroscopic or HPLC grade. Reagents were purchased from Sigma-Aldrich or Fluka and used without purification. $^1H$ and $^{13}C$ NMR spectra were recorded on a Bruker AC-400 spectrometer, operating at 400.13 MHz and 100.62 MHz, respectively. Chemical shifts are given in ppm (δ) relative to residual solvent peak for $^1H$ and $^{13}C$, coupling constants (J) are reported in Hertz, and the normal abbreviations are used. Thin layer chromatography (TLC) was performed using Merck silica gel plates (Kieselgel 60 $F_{254}$/0.2 mm thickness) and spots were visualised by UV light, then revealed by sulfuric acid-anisaldehyde spray followed by heating. Column chromatography was performed with Merck silica gel 60 (230-400 mesh). Preparative HPLC were carried out on a Perkin Elmer system (200 Pump) with a C18 reverse phase column (Kromasil, 5μ100Å, 150x4.5 mm) using a flow rate of 5.5 ml/min and a linear gradient of $CH_3CN$ (A) in 10 mM triethylammonium acetate buffer (B) at pH 7.5 over 20 min. Eluted products were visualized using a diode array detector. Purity of compounds was checked by anaytical HPLC on a C18 reverse phase column using a flow rate of 1 ml/min. ESI-TOF mass spectra were recorded by the mass spectroscopy laboratory (CNRS-ICSN, Gif-sur-Yvette).

**General procedure for *N*-alkylation (Scheme 2 and 3)**

To a stirred solution of thymine (or related base) (100 mg) in DMF (4 ml) was added anhydrous $K_2CO_3$ (100 mg). A solution of aryl halide (1.1 eq.) in DMF (1 ml) was added over a period of 1 h, the stirring was maintained overnight at room temperature. The insoluble was removed by filtration and the filtrate was concentrated to dryness by co-evaporation with xylene. The crude residue was dissolved in $CH_2Cl_2$ and washed with water. The organic layer was dried, concentrated under vacuum and purified by column chromatography on silica gel (gradient of methanol in dichloromethane) to give $N^1$,$N^3$-dialkylated and $N^1$-alkylated thymine in a 1:1.2-1.3 ratio.

Alternatively, alkylation of $N^3$-benzoyl-thymine (obtained in two steps from thymine in 74% yield according to the method described in "The Benzoylation of Uracil and Thymine", Cruickshank, K.A.; Jiricny, J.; Reese, C.B.; (1984)

*Tet. Letts.*, 25 (6), 681-684) afforded the $N^1$-alkylated $N^3$-benzoyl-thymine as a unique product in 84% yield. Benzoyl deprotection under basic conditions (33% aqueous ammonia in methanol) results in the corresponding $N^1$-alkylated thymine (nearly quant. yield).

**5-Methyl-1-(4-bromo-benzyl)-*1H*-pyrimidine-2,4-dione (11)**

Reaction of thymine (300 mg), $K_2CO_3$ (330 mg) with 4-bromobenzyl bromide (1.1 eq) in DMF (15 ml) yielded after purification compound **11** (302 mg, 43% yield). $^1H$ NMR ($CDCl_3$) δ: 1.96 (d, 3H, $CH_3$, J = 1.2 Hz), 4.85 (s, 2H, $PhCH_2$), 6.98 (d, 1H, H6, J = 1.2 Hz), 7.19 (d, 2H, H arom., J = 8 Hz), 7.51 (d, 2H, H arom.), 9.47 (bs, 1H, NH). $^{13}C$ NMR ($CDCl_3$) δ: 12.76 ($CH_3$), 50.86 ($PhCH_2$), 111.98 (C5), 122.94 (C arom.), 130.07 and 132.63 (CH arom.), 134.93 (C arom.), 139.88 (C6), 151.59 (C2), 164.53 (C4). MS (ESI-TOF) *m/z* 295.0 and 297.0 (15%, $M+H)^+$, 317.0 and 319.0 (20%, $M+Na)^+$, 339.0 and 341.0 (100%, 85%, $M+2Na)^+$.

***N*$^3$-Benzoyl-5-Methyl-1-(4-bromo-benzyl)-*1H*-pyrimidine-2,4-dione (11 bis)**

Reaction of $N^3$-benzoyl-thymine (230 mg, 1 mmol) with 4-bromobenzyl bromide (140 mg) yielded after purification compound **11bis** (336 mg, 84% yield). $^1H$ NMR (DMSO-d6) δ: 1.85 (d, 3H, $CH_3$, J = 1.1 Hz), 4.90 (s, 2H, $PhCH_2$), 7.32 (d, 2H, H arom., J = 8.4 Hz), 7.78 (m, 4H, H arom. and H arom. Bz), 7.78 (t, 1H, H arom. Bz), 7.93 (m, 2H, H arom. Bz), 7.95 (d, 1H, H6, J = 1.1 Hz). $^{13}C$ NMR (DMSO-d6) δ: 12.73 ($CH_3$), 51.14 ($PhCH_2$), 110.08 (C5), 121.93 (C arom.), 130.36, 130.71, 131.14 (CH arom.), 131.98 (C arom.), 132.49 and 136.32 (CH arom.), 136.64 (C arom.), 143.04 (C6), 150.38 (C2), 163.69 (C4), 170.46 (COBz). MS (ESI-TOF) *m/z* 421.0 and 423.0 (100% and 90%, M+Na).

**1-[4-(4-Hydroxy-but-1-ynyl)-benzyl]-5-methyl-1*H*-pyrimidine-2,4-dione (30)**

To a solution of compound 11 (1.94 g, 6.57 mmol) in dry $CH_2Cl_2$ (10 ml) were added under Ar freshly distilled $Et_3N$ (27 ml), 3-butyn-1-ol (0.46 g, 6.57 mmol), tetrakis(triphenylphosphine)palladium (0.45 g, 0.39 mmol) and cuprous iodide (25 mg, 0.13 mmol). The reaction mixture was heated under reflux for 72 h. To the cooled mixture, $CH_2Cl_2$ (100 ml) was added and the resulting solution was washed with 5% aqueous citric acid (3x100 ml), then water. The organic layer was dried over $Na_2SO_4$, concentrated under vacuo. Purification by silica gel column chromatography (0-10% gradient of methanol in dichloromethane) afforded recovered starting material (0.52 g, 27%), then compound **30** as a white powder (0.44 g, 24%). $^1H$ NMR (DMSO-d6) δ: 1.75 (d, 3H, $CH_3$, J = 1.2 Hz), 2.54 (t, 2H, $CH_2$), 3.58 (q, 2H, $CH_2OH$), 4.83 (s, 2H, $PhCH_2$), 4.92 (t, 1H, OH), 7.25 (d, 2H, H arom., J = 8 Hz), 7.37 (d, 2H, H arom., J = 8 Hz), 7.61 (d, 1H, H6, J = 1.2 Hz), 11.33 (bs, 1H, NH). $^{13}C$ NMR (DMSO-d6) δ: 12.77 ($CH_3$), 24.09 ($CH_2$), 50.72 ($PhCH_2$), 60.57 ($CH_2$), 81.54 (C alcyn), 89.68 (C alcyn), 109.98 (C5), 123.32 (C arom.), 128.40 (CH arom.), 132.38 (CH arom.), 137.63 (C arom.), 142.14 (C6), 151.84 (C2), 165.13 (C4). HRMS (ESI-TOF) *m/z* calculated for $C_{16}H_{17}N_2O_3$+Na 307.1059; found 307.1048.

**1-[4-(4-Hydroxy-butyl)-benzyl]-5-methyl-1*H*-pyrimidine-2,4-**dione (31)

To a solution of compound **30** (0.34 g, 1.19 mmol) in a mixture of $CH_2Cl_2$/methanol (1 ml/10 ml) was added Pd/C (100 mg). Hydrogen was applied overnight, then the mixture was passed through celite and the filtrate concentrated to dryness to give **31** after purification by silica gel column chromatography (0-10% gradient of methanol in dichloromethane) (0.24 g, 70% yield). $^1H$ NMR (DMSO-d6) δ: 1.42 (m, 2H, $\underline{CH_2}CH_2$), 1.57 (m, 2H, CH), 1.75 (d, 3H, $CH_3$, J = 1.2 Hz), 2.55 (t, 2H, $CH_2Ph$), 3.40 (m, 2H, $CH_2OH$), 4.36 (t, 1H, OH), 4.79 (s, 2H, $PhCH_2$), 7.11 (d, 2H, H arom., J = 8.4 Hz), 7.20 (d, 2H, H arom., J = 8.4 Hz), 7.61 (d, 1H, H6, J = 1.2 Hz), 11.30 (bs, 1H, NH). $^{13}C$ NMR (DMSO-d6) δ: 12.77 ($CH_3$), 28.27 ($CH_2$), 32.93 ($CH_2$), 35.47 ($CH_2$), 50.63 ($PhCH_2$), 61.36 ($CH_2$), 109.83 (C5), 128.29 (C arom.), 129.41 (CH arom.), 135.13 (CH arom.), 142.12 (C6), 142.67 (C arom.), 151.85 (C2), 165.09 (C4). HRMS (ESI-TOF) *m/z* calculated for $C_{16}H_{20}N_2O_3$+Na 311.1372; found 311.1374.

**4-[4-(5-Methyl-2,4-dioxo-3,4-dihydro-2*H*-pyrimidin-1-yl-methyl)-phenyl]-butyric acid (20)**

To a stirred solution of compound **31** (80 mg, 0.28 mmol) in dry $CH_2Cl_2$ (3 ml) were added *t*-butanol (0.54 ml, 5.6 mmol), acetic anhydride (0.26 ml, 2.8 mmol) and pyridinium dichromate (0.21 g, 0.56 mmol). After stirring for 1 h 30 at room temperature, starting material was consummed as judged by TLC. The crude mixture was loaded on a silica gel column chromatography conditionned in ethyl acetate, let on the silica gel for 15 min., then eluted with ethyl acetate. The fractions containing the expected ester were pooled, concentrated under vacuo and then treated with 2N NaOH (1ml) in methanol (5 ml) overnight. The reaction mixture was acidified by addition of cationic resin (Dowex $H^+$) until pH 3, filtered and lyophilized. Purification by reverse phase HPLC (5-30% linear gradient of acetonitrile in buffer, *R*t = 14 min.) gave compound **20** (20 mg, 25%). $^1H$ NMR (DMSO-d6) δ: 1.75 (d, 3H, $CH_3$, J = 1.2 Hz), 1.78 (m, 2H, $CH_2$), 2.20

(t, 2H, $CH_2$), 2.57 (t, 2H, $CH_2$), 4.80 (s, 1H, H5), 7.19 (m, 4H, H arom.), 7.61 (d, 1H, H6, J = 1.2 Hz), 11.30 (bs, 1H, NH). $^{13}C$ NMR (DMSO-d6) δ: 12.78 ($CH_3$), 27.12 ($CH_2$), 33.97 ($CH_2$), 34.90 ($CH_2$), 50.61 ($PhCH_2$), 109.83 (C5), 128.37 (CH arom.), 129.44 (CH arom.), 135.37 (C arom.), 141.92 (C arom.), 142.12 (C6), 151.86 (C2), 165.08 (C4), 175.09 (COOH). MS (ESI-TOF) *m*/*z* 325.1 (100%, M+Na)$^+$. HRMS (ESI-TOF) *m*/*z* calculated for $C_{16}H_{18}N_2O_4$+Na 325.1178; found 325.0357.

**1-(4-Bromo-benzyl)-*1H*-pyrimidine-2,4-dione (35)**

A suspension of uracil (4.94 g, 44.0 mmol) and potassium carbonate (4.7 g, 33.9 mmol) in anhydrous DMF (60 ml) was stirred at room temperature for 1 h, then 4-bromobenzylmethyl bromide (8.47 g, 33.9 mmol) was added. After stirring overnight, the mixture was purified to give compound **35** (3.15 g, 33% yield). $^1H$ NMR (DMSO-d6) δ: 4.85 (s, 2H, $PhCH_2$), 5.60 (d, 1H, H5, J = 7.8 Hz), 7.26 (d, 2H, H arom., J = 8.4 Hz), 7.60 (dt, 2H, H arom., J = 8.4 Hz, J = 2 Hz), 7.76 (d, 1H, H6, J = 7.8 Hz), 11.34 (bs, 1H, NH). $^{13}C$ NMR (DMSO-d6) δ: 50.60 ($PhCH_2$), 102.32 (C5), 121.69 (C arom.), 130.58 CH arom.), 132.39 (CH arom.), 137.17 (CH arom.), 146.38 (C6), 151.84 (C2), 164.48 (C4). MS (ESI-TOF) *m*/*z* 281.0 and 283.0 (M+H)$^+$, 303.0 and 305.0 (M+Na)$^+$, 325.0 and 327.0 (M+2Na)$^+$, 335.0 and 337.0.

**1-(4-Hydroxy-but-1-ynyl-benzyl)-*1H*-pyrimidine-2,4-dione (36)**

To a solution of compound 35 (1.46 g, 5.21 mmol) in dry $CH_2Cl_2$ (40 ml) were added under Ar freshly distilled $Et_3N$ (45 ml), 3-butyn-1-ol (0.44 g, 6.57 mmol), tetrakis(triphenylphosphine)palladium (0.37 g, 0.31 mmol) and cuprous iodide (20 mg, 0.10 mmol). The reaction mixture was heated ay 80°C for 90 h and worked up as for compound **30** to give compound **36.** $^1H$ NMR (DMSO-d6) δ: 1.19 (t, 21H, $CH_3$), 2.54 (t, 2H, $CH_2$), 3.10 (t, 14H, $CH_2$), 3.57 (q, 2H, $CH_2$), 4.87 (s, 2H, $PhCH_2$), 4.89 (t, 1H, OH), 5.60 (dd, 1H, H5, J = 7.8 Hz, J = 2.1 Hz), 7.25 (d, 2H, H arom., J = 8.3 Hz), 7.38 (dd, 2H, H arom., J = 6.5 Hz, J = 2.7 Hz), 7.76 (d, 1H, H6), 9.10 (bs, 2.3H, NH), 11.33 (bs, 1H, NH). $^{13}C$ NMR (DMSO-d6) δ: 24.12 ($CH_2$), 50.88 ($PhCH_2$), 60.56 ($CH_2$), 81.52 (C alcyn) , 89.72 (C alcyn), 102.26 (C5), 123.36 (C arom.), 128.41 (CH arom.), 132.38 (CH arom.), 137.50 (C arom.), 146.38 (C6), 151.85 (C2), 164.50 (C4). MS (ESI-TOF) *m*/*z* 293.1 (100%, M+Na)$^+$, 333.2 (5%, M+Na+K)$^+$.

**1-[4-(4-Hydroxy-butyl)-benzyl]-1*H*-pyrimidine-2,4-dione (37)**

Compound **36** (0.55g, 2.0 mmol) was hydrogenated as for compound 30 to give after purification compound 37 (0.25 g, 46%). $^1H$ NMR (DMSO-d6) δ: 1.41 (m, 2H, $CH_2$), 1.57 (m, 2H, $CH_2$), 2.55 (t, 2H, $CH_2$), 3.39 (m, 2H, $CH_2$), 4.35 (t, 1H, OH), 4.83 (s, 2H, $PhCH_2$), 5.58 (dd, 1H, H5, J = 7.8 Hz, J = 2.3 Hz), 7.19 (m, 4H, H arom.), 7.74 (d, 1H, H6, J = 7.8 Hz), 11.30 (bs, 1H, NH). $^{13}C$ NMR (DMSO-d6) δ: 28.26 ($CH_2$), 32.93 ($CH_2$), 35.46 ($CH_2$), 50.84 ($PhCH_2$), 61.34 ($CH_2OH$), 102.14 (C5), 128.40 (CH arom.), 129.42 (CH arom.), 134.95 (C arom.), 142.74 (C arom.), 146.45 (C6), 151.86 (C2), 164.50 (C4). MS (ESI-TOF) *m*/*z* 297.1 (100%, M+Na)$^+$, 431.4 (40%).

**4-[4-(2,4-dioxo-3,4-dihydro-2*H*-pyrimidin-1-ylmethyl)-phenyl]-butyric acid (38)** Compound **38** was obtained in two steps : chromic oxidation of compound 37 (135 mg, 0.5 mmol) as described for compound 32 gave the corresponding t-butyl ester (125 mg, 70%). Saponification of the ester (105 mg, 0.29 mmol) afforded compound **38** as a white powder (68 mg, 80%). $^1H$ NMR (DMSO-d6) δ: 1.77 (m, 2H, $CH_2$), 2.21 (t, 2H, $CH_2$), 2.57 (t, 2H, $CH_2$), 4.83 (s, 2H, $PhCH_2$), 5.58 (dd, 1H, H5, J = 2.2 Hz and J = 7.8 Hz), 7.20 (m, 4H, H arom.), 7.74 (d, 1H, H6, J = 8.8 Hz), 11.30 (bs, 1H, NH), 12.05 (bs, 1H, COOH). $^{13}C$ NMR (DMSO-d6) δ: 27.08 ($CH_2$), 33.91 ($CH_2$), 34.89 ($CH_2$), 50.83 ($PhCH_2$), 102.15 (C5), 128.40 (CH arom.), 129.46 (CH arom.), 135.19 (C arom.), 141.97 (C arom.), 146.44 (C6), 151.86 (C2), 164.50 (C4), 175.04 (COOH). HRMS (ESI-TOF) *m*/*z* calculated for $C_{15}H_{16}N_2O_4$+Na 311.1008; found 311.1022.

**4-(5-Bromo-2,4-dioxo-3,4-dihydro-2*H*-pyrimidin-1-ylmethyl)-butyric acid (21)**

To compound **38** (50 mg, 0.17 mmol) in pyridine (3 ml) was added a 1M solution of bromine in $CCl_4$ (0.23 ml). After stirring for 1h30 reaction was complete, the solution was concentrated under vacuo. Purification by silica gel column chromatography (0-20% gradient of methanol in dichloromethane) afforded compound **21** as a pale yellow powder (45 mg, 70%). $^1H$ NMR (DMSO-d6) δ: 1.77 (m, 2H, $CH_2$), 2.21 (t, 2H, $CH_2$, J = 7.3 Hz), 2.57 (t, 2H, $CH_2$), 4.84 (s, 2H, $PhCH_2$), 7.18 (d, 2H, H arom., J = 8.1 Hz), 7.25 (d, 2H, H arom.), 8.35 (d, 1H, H6), 11.83 (bs, 1H, NH), 12.05 (bs, 1H, COOH). $^{13}C$ NMR (DMSO-d6) δ: 27.08 ($CH_2$), 33.92 ($CH_2$), 34.90 ($CH_2$), 51.32 ($PhCH_2$), 95.92 (C5), 128.51 (CH arom.), 129.45 (CH arom.), 134.84 (C arom.), 142.09 (C arom.), 146.02 (C6), 151.22 (C2), 160.44 (C4), 175.04 (COOH). MS (ESI-TOF) *m*/*z* 389.0 (100%, M+Na)$^+$, 391.0 (86%, M+Na)$^+$. HRMS (ESI-TOF) *m*/*z* calculated for $C_{15}H_{15}N_2O_4{}^{79}Br$+Na 389.0113 and $C_{15}H_{15}N_2O_4{}^{81}Br$+Na 391.0092; found 389.0140 (100%) and 391.0136 (87%).

**5-Methyl-1-Benzyl-*1H*-pyrimidine-2,4-dione (3)**

Reaction of thymine with benzyl bromide yielded after purification compound 3 (57 mg, % yield). $^1H$ NMR ($CDCl_3$) δ: 1.90 (d, 3H, $CH_3$, J = 1.1 Hz), 4.92 (s, 2H, $PhCH_2$), 7.00 (d, 1H, H6, J = 1.1 Hz), 7.31 (m, 2H, H arom.), 7.39 (m, 3H, H arom.), 9.14 (bs, 1H, NH). MS (ESI-TOF) *m*/*z* 217.1 (60%, $M+H)^+$, 239.1 (100%, $M+Na)^+$.

**5-Bromo-1-benzyl-*1H*-pyrimidine-2,4-dione (4)**

Reaction of 5-bromo-uracil with benzyl bromide yielded after purification compound 4. $^1H$ NMR ($CDCl_3$) δ: 4.91 (s, 2H, $PhCH_2$), 7.30-7.40 (m, 5H, H arom.), 8.37 (s, 1H, H6), 11.84 (bs, 1H, NH).

**5-Fluoro-1-benzyl-*1H*-pyrimidine-2,4-dione (5)**

Reaction of 5-fluoro-uracil with benzyl bromide yielded after purification compound 5. $^1H$ NMR (DMSO-d6) δ: 4.89 (s, 2H, $PhCH_2$), 7.34 (m, 5H, H arom.), 8.37 (s, 1H, H6), 11.84 (bs, 1H, NH). MS (ESI-TOF) *m*/*z* 279 (M+2Na).

**5-Methyl-1-(4-fluoro-benzyl)-*1H*-pyrimidine-2,4-dione (6)**

Reaction of thymine with 4-fluoro-benzyl chloride yielded after purification compound 6 (64 mg, 35% yield). $^1H$ NMR ($CDCl_3$) δ: 1.89 (d, 3H, $CH_3$, J = 1.1 Hz), 4.87 (s, 2H, $PhCH_2$), 6.99 (d, 1H, H6, J = 1.1 Hz), 7.06 (m, 2H, H arom.), 7.30 (m, 2H, H arom.), 9.75 (bs, 1H, NH). $^{13}C$ NMR ($CDCl_3$) δ: 12.75 ($CH_3$), 50.76 ($PhCH_2$), 111.87 (C5), 116.18 and 116.52 (CH arom.), 130.25 and 130.33 (CH arom.), 131.78 and 131.81 (C arom.), 139.95 (C6), 151.73 (C2), 161.84 and 161.30 (C arom.), 164.74 (C4). MS (ESI-TOF) *m*/*z* 235.1 (90%, $M+H)^+$, 257.1 (40%, $M+Na)^+$, 279.1 (50%, $M+2Na)^+$.

**5-Methyl-1-(3-fluoro-benzyl)-*1H*-pyrimidine-2,4-dione (7)**

Reaction of thymine with 3-fluoro-benzyl chloride yielded after purification compound 7 (48 mg, 26% yield). $^1H$ NMR ($CDCl_3$) δ: 1.90 (d, 3H, $CH_3$, J = 1.1 Hz), 4.90 (s, 2H, $PhCH_2$), 7.05 (m, 3H, H6 and H arom.), 7.08 (d, 1H, H arom.), 7.35 (m, 1H, H arom.), 9.75 (bs, 1H, NH). $^{13}C$ NMR ($CDCl_3$) δ: 12.75 ($CH_3$), 50.86 ($PhCH_2$), 111.98 (C5), 115.18 and 115.40 (CH arom.), 115.69 and 115.90 (CH arom.), 123.83 and 123.86 (CH arom.), 131.06 and 131.14 (CH arom.), 138.36 and 138.44 (C arom.), 140.00 (C6), 151.68 (C2), 162.19 (C4), 164.66 and 164.72 (C arom.). MS (ESI-TOF) *m*/*z* 235.1 (100%, $M+H)^+$, 257.1 (5%, $M+Na)^+$, 279.1 (70%, $M+2Na)^+$.

**5-Methyl-1-(3,4-difluoro-benzyl)-*1H*-pyrimidine-2,4-dione (8)**

Reaction of thymine with 3,4-difluoro-benzyl bromide yielded after purification compound **8** (60 mg, 30% yield). $^1H$ NMR ($CDCl_3$) δ: 1.89 (d, 3H, $CH_3$, J = 1.1 Hz), 4.90 (s, 2H, $PhCH_2$), 5.30 (d, 1H, =CH, J = 11 Hz), 5.77 (d, 1H, =CH, J = 18 Hz), 6.72 (dd, 1H, =CH, J = 11 Hz, J = 18 Hz), 6.98 (d, 1H, H6, J = 1.1 Hz), 7.27 (m, 2H, H arom., J = 8 Hz), 7.42 (m, 2H, H arom.), 9.26 (bs, 1H, NH). $^{13}C$ NMR ($CDCl_3$) δ: 12.74 ($CH_3$), 50.61 ($PhCH_2$), 112.15 (C5), 117.47 and 117.64 (CH arom.), 118.25 and 118.43 (CH arom.), 124.46, 124.49, 124.52 and 124.56 (CH arom.), 132.85 and 132.90 (C arom.), 139.76 (C6), 149.46, 149.58, 149.63 and 149.75 (C arom.), 151.49 (C2), 151.93, 152.06, 152.11 and 152.24 (C arom.), 164.48 (C4). MS (ESI-TOF) *m*/*z* 253.1 (100%, $M+H)^+$.

**5-Methyl-1-(4-chloro-benzyl)-*1H*-pyrimidine-2,4-dione (9)**

Reaction of thymine with 4-chloro-benzyl chloride (1.1 eq) yielded after purification compound **8** (71 mg, 36% yield). $^1H$ NMR ($CDCl_3$) δ: 1.89 (d, 3H, $CH_3$, J = 1 Hz), 4.86 (s, 2H, $PhCH_2$), 6.99 (d, 1H, H6), 7.25 (d, 2H, H arom.), 7.34 (d, 2H, H arom.), 9.88 (bs, 1H, NH). $^{13}C$ NMR ($CDCl_3$) δ: 12.75 ($CH_3$), 50.80 ($PhCH_2$), 111.96 (C5), 126.65 and 129.77 (CH arom.), 134.44 and 134.82 (C arom.), 139.92 (C6), 151.68 (C2), 164.66 (C4). MS (ESI-TOF) *m*/*z* 251.1 (20%, $M+H)^+$, 295.0 (100%, $M+2Na)^+$.

**5-Methyl-1-(3-chloro-benzyl)-*1H*-pyrimidine-2,4-dione (10)**

Reaction of thymine with 3-chloro-benzyl chloride (1.1 eq) yielded after purification compound (52 mg, 26% yield). $^1H$ NMR ($CDCl_3$) δ: 1.92 (d, 3H, $CH_3$, J = 1.1 Hz), 4.88 (s, 2H, $PhCH_2$), 6.99 (d, 1H, H6, J = 1.1 Hz), 7.20 (m, 1H, H arom.), 7.28 (m, 1H, H arom.), 7.32 (m, 2H, H arom.), 9.45 (bs, 1H, NH). $^{13}C$ NMR ($CDCl_3$) δ: 12.77 ($CH_3$), 50.85 ($PhCH_2$), 112.03 (C5), 126.44, 128.37, 129.06 and 130.79 (CH arom.), 135.36 (C arom.), 137.92 (C6), 139.92 (C arom.), 151.55 (C2), 164.52 (C4). MS (ESI-TOF) *m*/*z* 251.1 (100%, $M+H)^+$, 273.0 (25%, $M+Na)^+$, 295.0 (35%, $M+2Na)^+$.

**5-Methyl-1-(4-bromo-benzyl/-*1H*-pyrimidine-2,4-dione (11)**

Reaction of thymine (300 mg), $K_2CO_3$ (330 mg) with 4-bromobenzyl bromide (1.1 eq) in DMF (15 ml) yielded after purification compound (302 mg, 43% yield).

$^1H$ NMR (DMSO-d6) δ: 1.75 (d, 3H, $CH_3$, J = 1.2 Hz), 4.81 (s, 2H, $PhCH_2$), 7.26 (d, 2H, H arom., J = 8.3 Hz), 7.56 (d, 2H, H arom.,J = 8.3 Hz), 7.64 (d, 1H, H6, J = 1.2 Hz), 11.34 (bs, 1H, NH). $^{13}C$ NMR (DMSO-d6) δ: 12.79 ($CH_3$), 50.37 ($PhCH_2$), 109.99 (C5), 121.63 (C arom.), 130.57 and 132.38 (CH arom.), 137.34 (C arom.), 142.03 (C6), 151.83 (C2), 165.07 (C4). MS (ESI-TOF) *m/z* 295.0 and 297.0 (15%, M+H)$^+$, 317.0 and 319.0 (20%, M+Na)$^+$, 339.0 and 341.0 (100%, 85%, M+2Na)$^+$.

**B- Biological Activity**

Activity was determined using the coupled spectrophotometric assay described in Blondin *et al.* (C. Blondin, L. Serina, L. Wiesmüller, A.-M. Gilles, O. Bârzu, *Anal. Biochem.* 1994, *220,* 219).

$$\text{ATP + dTMP} \xrightarrow{\text{TMPK}} \text{ADP + dTDP}$$

$$\text{dTDP + ATP} \xrightarrow{\text{NDPK}} \text{ADP + dTTP}$$

$$\text{2 ADP + 2 PEP} \xrightarrow{\text{PK}} \text{2 ATP + 2 pyruvate}$$

$$\text{2 pyruvate + 2 NADH} \xrightarrow{\text{LDH}} \text{2 lactate + 2 NAD}^+$$

Each mole of transferred phosphoryl group generates two moles of NAD+ and the decrease in absorbance at 334 nm is followed in an Eppendorf ECOM 6122 photometer. The reaction medium (0.5 ml final volume) contained 50 mM Tris-HCl pH 7.4, 50 mM KCl, 2 mM $MgCl_2$, 0.2 mM NADH, 1 mM phosphoenol pyruvate and 2 units each of lactate dehydrogenase, pyruvate kinase and nucleoside diphosphate kinase. One unit of enzyme activity corresponds to 1 μmole of the product formed in 1 min. at 30°C and pH 7.4. The concentrations of ATP and dTMP were kept constant at 0.5 mM and 0.05 mM respectively, whereas the concentrations of analogues varied between 0.005 and 8 mM. Equation 1 was used to calculate the Ki values using Equations 2 and 3 (classical competitive inhibition model following the Lineweaver-Burk representation):

$$K_i = \frac{K_m\ [I]}{\left(\frac{v}{v_i} - 1\right)\left(K_m + [S]\right)} \quad \text{(Eq.1)}$$

$$v = \frac{V_m\ [S]}{[S] + K_m} \quad \text{(Eq.2)}$$

$$v_i = \frac{V_m [S]}{[S] + K_m \left(1 + \frac{[I]}{K_i}\right)} \quad \text{(Eq.3)}$$

where v and $v_i$ are the reaction velocities respectively in the absence and in the presence of the analogue at a concentration value [I]; Km is the Km for dTMP (4.5 μM for TMPKmt and 5 μM for TMPKh); [S] is the concentration of dTMP (50 μM).

The results are the exposed in Table 1:

Table 1: Biological activity of the molecules **1** to **22.**

| Compounds | TMPKmt W1002 |
|---|---|
| | Ki (μM) |
| dTMP | Km = 4.5 |
| dT | 27 |
| 1 | 110 |
| 2 | 68 |
| 3 | 75 |
| 4 | 44 |
| 5 | N.A. |
| 6 | 45 |
| 7 | 90 |
| 8 | 67 |
| 9 | 50 |
| 10 | 44 |
| 11 | 38 |
| 12 | 980 |
| 13 | 810 |
| 14 | 240 |
| 15 | N.A. |
| 16 | N.A. |
| 17 | N.A. |
| 18 | N.A. |
| 19 | 265 |
| 20 | 16.5 |
| 21 | 12.3 |
| 22 | 32 |

## Claims

1. Molecule responding to formula (I):

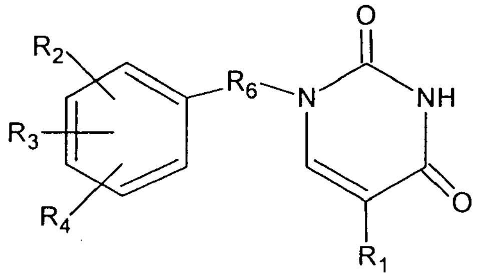

**(I)**

wherein:

- $R_1$ is selected from the group consisting of: $CH_3$, $-CF_3$, a halogen atom, $-NH_2$, -COOH, $-CONH_2$,
- $R_2$, $R_3$, $R_4$, identical or different, are selected from the group consisting of: H, a halogen atom, $C_1$-$C_8$ alkyl, $C_2$-$C_8$ alkenyl, $C_2$-$C_8$ alkynyl, -OH, - $NH_2$, -COOH, $-CONH_2$, -CN, $-COOR_5$, $-COR_5$, $-OR_5$, substituted $C_1$-$C_8$ alkyl , substituted $C_2$-$C_8$ alkenyl, or substituted $C_2$-$C_8$ alkynyl wherein the substituent is selected from the group consisting of: -OH, $-NH_2$, -COOH, $-CONH_2$, -CN, $-COOR_5$, - $COR_5$, $-OR_5$, a halogen atom;
- $R_5$ is selected from the group consisting of $C_1$-$C_6$ alkyl;
- $R_6$ is selected among: $C_1$-$C_4$ alkyl, $C_2$-$C_4$ alkenyl, carbonyl (=C=O), $-(CF_2)n-$
- n is an integer selected from 1, 2, 3,

and their pharmaceutically acceptable salts.

**2.** Molecule according to claim 1, wherein one or more of the following conditions is satisfied:

- $R_6$ is $-CH_2-$;
- $R_1$ is selected from the group consisting of: $-CH_3$, -Br, -Cl;
- at least one group among $R_2$, $R_3$, $R_4$ is H.

**3.** Molecule according to any one of the preceding claims, wherein $R_2 = R_3 = H$, $R_4$ is in the para position on the phenyl ring and $R_4$ is selected from the group consisting of substituted $C_1$-$C_6$ alkyl or substituted $C_2$-$C_6$ alkenyl, wherein the substituent is -COOH.

**4.** Molecule according to any one of the preceding claims, wherein it is selected from the group consisting of:

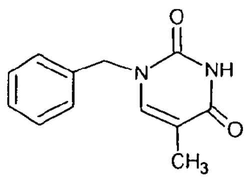

**3**

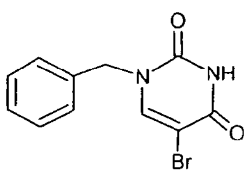

**4**

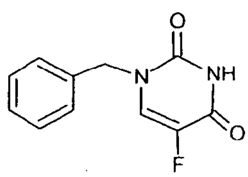

**5**

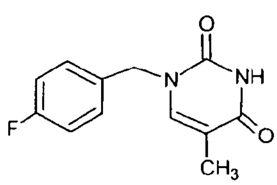

**6**

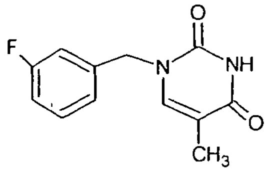 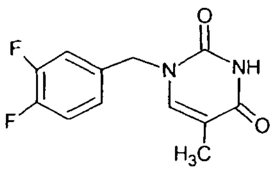 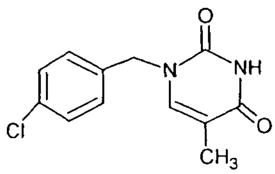 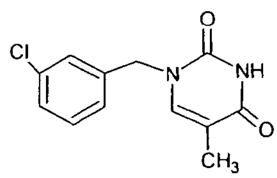

7 8 9 10

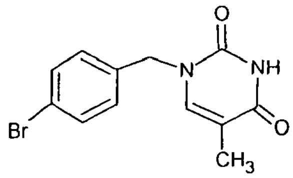 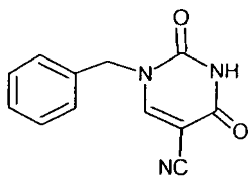 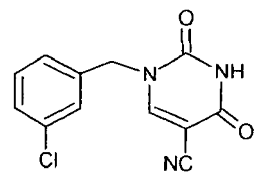

11 12 13

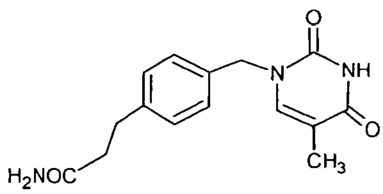 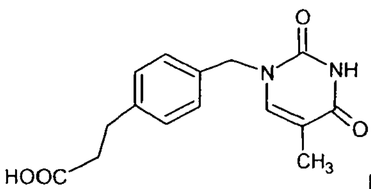 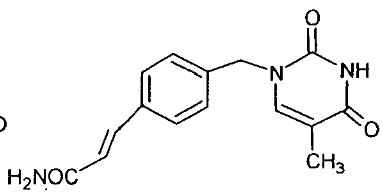

1 2 14

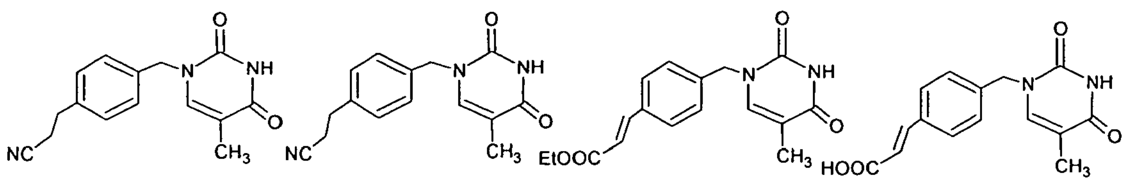

15 16 17 18

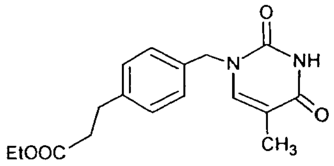

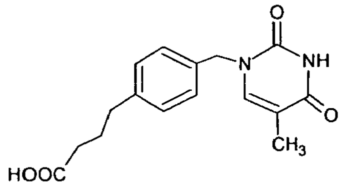

**19** **20**

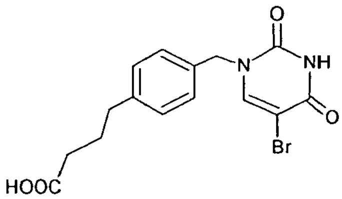

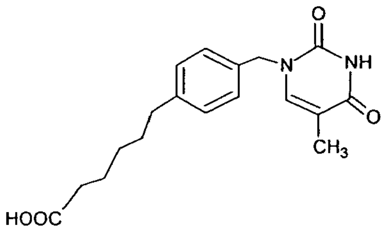

**21** **22**

5. Molecule according to anyone of claim 1 to 4, **characterized in that** it is selected from the group consisting of:

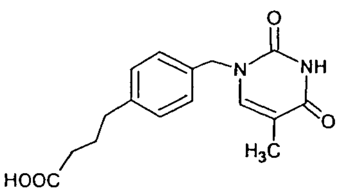

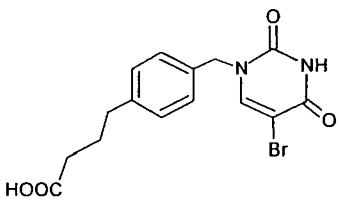

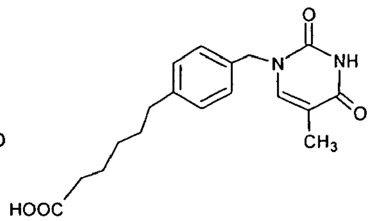

**20** **21** **22**

6. Pharmaceutical composition comprising at least one compound of formula (I) according to anyone of the preceding claims in a pharmaceutically acceptable carrier.

7. Use of a molecule of formula (I) according to anyone of the preceding claims for the preparation of a medicament for the prevention and/or treatment of a pathology caused by mycobacteria.

8. Use according to claim 7, for the prevention and/or treatment of tuberculosis.

9. Use according to claim 7, for the prevention and/or treatment of leprosy.

10. Use according to anyone of the preceding claims, wherein the daily dose of active principle is comprised between 0,1 and 500 mg/kg.

11. Use of a molecule of formula (I) according to anyone of claims 1 to 5 as an inhibitor of a mycobacteria TMPK *in vitro.*

12. Use according to claim 11, as an inhibitor of *M. tuberculosis* TMPK *in vitro.*

**13.** Process for the preparation of a molecule responding to formula (I) according to anyone of claim 1 to 5, **characterized in that**:

- A haloaryl of formula (II) is reacted with a thymine or thymine derivative or uracyle or uracyle derivative of formula (III) to give the condensate (IV),

wherein X represents a halogen atom, preferentially Br; $X_2$, $X_3$, $X_4$ are selected among $R_2$, $R_3$ and $R_4$ respectively and their chemical precursors, $X_1$ is selected among $R_1$ and its chemical precursors, $X_5$ is selected among H and the benzyl group (Bzl);

- In a second step and if necessary, $X_1$, $X_2$, $X_3$, $X_4$ and $X_5$ are transformed into $R_1$, $R_2$, $R_3$, $R_4$ and H respectively to give the molecule of formula (I)

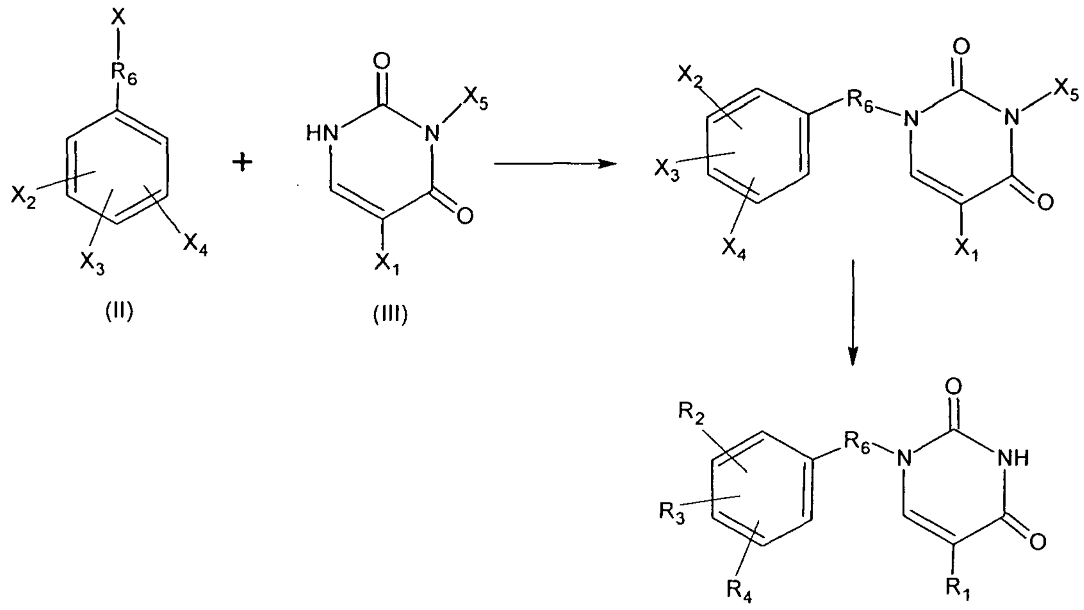

(II) (III) (IV) (I)

**Scheme 1**

**14.** Molecule responding to formula (V):

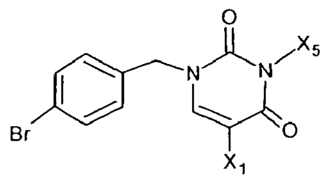

(V)

wherein $X_1$ is selected among $R_1$ and its chemical precursors,
$R_1$ is selected from the group consisting of: $CH_3$, $-CF_3$, a halogen atom, $-NH_2$, $-COOH$, $-CONH_2$.
and
$X_5$ is selected among H and the benzyl group (Bzl).

**15.** Molecule according to claim 14, **characterized in that** it responds to formula **11** or to formula **11bis**:

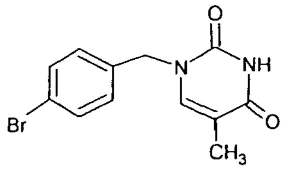

11

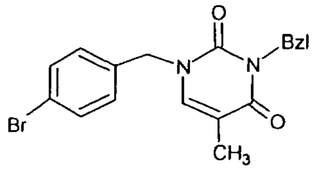

11bis

European Patent Office

# EUROPEAN SEARCH REPORT

Application Number
EP 04 29 2629

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X<br><br><br><br>A | EP 0 748 800 A (F. HOFFMANN-LA ROCHE AG) 18 December 1996 (1996-12-18)<br>* page 21, line 3 *<br>* page 21, line 20 - line 21 *<br>* page 22, line 59; example 5 *<br>----- | 1,2,4<br><br><br><br>13-15 | C07D239/54<br>A61K31/513<br>A61P31/06<br>A61P31/08 |
| X | WO 02/061110 A (BIO-RAD LABORATORIES, INC; SEGEV, DAVID) 8 August 2002 (2002-08-08)<br>* figure 5 (i), compound 31 *<br>----- | 1,2 | |
| X | DE 19 59 705 A1 (FARBENFABRIKEN BAYER AG) 3 June 1971 (1971-06-03)<br>* page 8, formula on the bottom of right-hand column *<br>----- | 1,2 | |
| X | Y. AOYAMA ET AL: "Preparation of an intramolecular adenine-thymine base pair" TETRAHEDRON LETTERS, vol. 31, no. 8, 1990, pages 1177-1180, XP002334422<br>* page 1178, compound 4 *<br>----- | 1,2 | TECHNICAL FIELDS SEARCHED (Int.Cl.7) |
| X | PATENT ABSTRACTS OF JAPAN vol. 1997, no. 04, 30 April 1997 (1997-04-30) & JP 08 325238 A (ASAHI GLASS CO LTD), 10 December 1996 (1996-12-10)<br>* formula III *<br>* abstract *<br>----- | 1 | C07D |

-/--

The present search report has been drawn up for all claims

1

| Place of search | Date of completion of the search | Examiner |
|---|---|---|
| Berlin | 4 July 2005 | Hass, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 04 29 2629

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A,D | POCHET S ET AL: "COMPARATIVE STUDY OF PURINE AND PYRIMIDINE NUCLEOSIDE ANALOGUES ACTING ON THE THYMIDYLATE KINASES OF MYCOBACTERIUM TUBERCULOSIS AND OF HUMANS" CHEMBIOCHEM - A EUROPEAN JOURNAL OF CHEMICAL BIOLOGY, WILEY VCH, WEINHEIM, DE, vol. 4, no. 8, 4 August 2003 (2003-08-04), pages 742-747, XP001172838 ISSN: 1439-4227 * page 743, table 1; page 745, table 2 * ----- | 1,6-12 | |
| A | US 2003/229106 A1 (KALLA RAO ET AL) 11 December 2003 (2003-12-11) * paragraph [0756] - paragraph [0757] * * paragraph [0817] - paragraph [0818] * ----- | 1 | |

TECHNICAL FIELDS SEARCHED (Int.Cl.7)

The present search report has been drawn up for all claims

| Place of search | Date of completion of the search | Examiner |
|---|---|---|
| Berlin | 4 July 2005 | Hass, C |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 04 29 2629

This annex lists the patent family members relating to the patent documents cited in the above-mentioned European search report.
The members are as contained in the European Patent Office EDP file on
The European Patent Office is in no way liable for these particulars which are merely given for the purpose of information.

04-07-2005

| Patent document cited in search report | Publication date | Patent family member(s) | Publication date |
|---|---|---|---|
| EP 0748800 A | 18-12-1996 | AT 201016 T | 15-05-2001 |
| | | AU 710754 B2 | 30-09-1999 |
| | | AU 5469096 A | 19-12-1996 |
| | | BR 9602705 A | 08-09-1998 |
| | | CA 2178548 A1 | 10-12-1996 |
| | | CN 1149051 A ,C | 07-05-1997 |
| | | CZ 9601696 A3 | 15-01-1997 |
| | | DE 69612698 D1 | 13-06-2001 |
| | | DE 69612698 T2 | 06-12-2001 |
| | | DK 748800 T3 | 27-08-2001 |
| | | EP 0748800 A2 | 18-12-1996 |
| | | ES 2157366 T3 | 16-08-2001 |
| | | GR 3036307 T3 | 31-10-2001 |
| | | HK 1013065 A1 | 25-01-2002 |
| | | HU 9601529 A2 | 28-09-1998 |
| | | IL 118519 A | 22-12-1999 |
| | | JP 2721147 B2 | 04-03-1998 |
| | | JP 9100269 A | 15-04-1997 |
| | | MA 23899 A1 | 31-12-1996 |
| | | NO 962412 A | 10-12-1996 |
| | | NZ 286720 A | 26-08-1998 |
| | | PL 314635 A1 | 23-12-1996 |
| | | PT 748800 T | 30-10-2001 |
| | | RU 2175322 C2 | 27-10-2001 |
| | | SG 45486 A1 | 16-01-1998 |
| | | TR 970073 A2 | 21-02-1997 |
| | | ZA 9604561 A | 09-12-1996 |
| WO 02061110 A | 08-08-2002 | CA 2436665 A1 | 08-08-2002 |
| | | EP 1363640 A2 | 26-11-2003 |
| | | WO 02061110 A2 | 08-08-2002 |
| | | JP 2004537503 T | 16-12-2004 |
| | | US 2003191074 A1 | 09-10-2003 |
| DE 1959705 A1 | 03-06-1971 | NONE | |
| JP 08325238 A | 10-12-1996 | NONE | |
| US 2003229106 A1 | 11-12-2003 | US 2003139428 A1 | 24-07-2003 |
| | | US 2004176399 A1 | 09-09-2004 |
| | | US 2005101778 A1 | 12-05-2005 |
| | | US 2005038045 A1 | 17-02-2005 |
| | | AU 2002359365 A2 | 26-05-2003 |
| | | CA 2466477 A1 | 22-05-2003 |
| | | CN 1585769 A | 23-02-2005 |
| | | EP 1444233 A2 | 11-08-2004 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 04 29 2629

This annex lists the patent family members relating to the patent documents cited in the above-mentioned European search report.
The members are as contained in the European Patent Office EDP file on
The European Patent Office is in no way liable for these particulars which are merely given for the purpose of information.

04-07-2005

| Patent document cited in search report | Publication date | Patent family member(s) | Publication date |
|---|---|---|---|
| US 2003229106 A1 | | HU 0401925 A2 | 28-01-2005 |
| | | JP 2005509036 T | 07-04-2005 |
| | | WO 03042214 A2 | 22-05-2003 |

EPO FORM P0459

For more details about this annex : see Official Journal of the European Patent Office, No. 12/82